# EUROPEAN PATENT APPLICATION

(11) **EP 1 319 410 A1**
(43) Date of publication of application: **18.06.2003**
(21) Application number: 01129563.1
(22) Date of filing: 11.12.2001
(51) Int. Cl.: A61K 47/48

(54) **Use of micro-organisms for a directed delivery of substances to specific parts of the gut**

(71) Applicant: Société des Produits Nestlé S.A., 1800 Vevey (CH)
(72) Inventor: The designation of the inventor has not yet been filed
(74) Representative: Becker Kurig Straus

(57) **Abstract**

The present invention pertains to the use of micro-organisms, in particular lactic acid bacteria as e.g. Lactobacilli and Bifidobacteria for delivering substances beneficial to the recipient to specific parts of its gastro-intestinal tract. In particular, the present invention relates to an ingestable carrier containing micro-organisms with such properties and to the use thereof for supporting the well-being of individuals or for the treatment and/or prophylaxis of diseases.

## Description

The present invention pertains to the use of micro-organisms, in particular Lactobacilli and Bifidobacteria, for delivering substances beneficial to the recipient to specific predetermined parts of its gastro-intestinal tract. In particular, the present invention relates to an ingestable carrier containing micro-organisms designed to lyse at particular locations of the gut and to the use thereof for supporting the well-being of individuals or for the treatment and/or prophylaxis of diseases.

The gastro-intestinal tract is the major organ for taking up material to be utilized by the individual for maintaining its integrity. Such material comprises any source of energy, such as proteins, lipids or carbohydrates but also essential components, such as vitamins, essential oils etc.. In order to fulfill its task, the gastro-intestinal tract is designed to perform at different locations thereof different duties. E.g. in the stomach the prevailing acidic pH supports an early breakdown of digested material while in the intestine, the material will be further degraded by enzymes secreted by present bacteria and will eventually be resorbed.

The intestinal tract is colonized by micro-organisms, such as Bacteroides, Lactobacilli, Bifidobacteria and also E.coli, one of the major tasks thereof being to protect the individual from an attack by pathogens. This is mainly achieved by a competition with the pathogens for adherence sites and eventually for a location for growth. These bacteria also support the further degradation of the food material ingested by the individual.

In the past, probiotic organisms have attracted a great deal of attention in that some strains have been found to exhibit valuable properties to man and animals upon ingestion. Probiotics are considered to be viable microbes which promote the individual's health by preserving the natural microflora in the intestine. Probiotics may attach to the gastro-intestinal mucosa, at least transiently colonizing the gastro-intestinal tract and likewise preventing attachment of harmful microorganisms thereon. A crucial prerequisite for their action resides in that they have to reach the gut mucosa in a proper and viable form and do not get destroyed in the upper part of the gastro-intestinal tract, especially by the influence of the low pH prevailing in the stomach. In particular, some strains of the genus Lactobacillus or Bifidobacterium have been found to survive the acidic pH of the gastric environment upon passage and be able to colonize the intestinal mucosa, with their temporary or sustained maintenance in the gut having numerous positive effects on the health of the individuals having incorporated them. These beneficial effects have by and large been attributed to specific compounds/metabolites that the probiotics produce and secrete during their stay in the gut.

In this respect, EP 0 577 903 discloses lactic acid bacteria having the ability of deplacing *Helicobacter pylori* from gastric cells, the acknowledged cause for the development of gasric ulcer and cancer. The lactic acid bacteria utilized in this respect are deemed to release some compounds detrimental to the growth of Helicobacter so that a subsequent or excessive proliferation of these pathogens is reduced or even prevented.

WO 97/00078 discloses a specific strain, termed Lactobacillus rhamnosus GG (ATCC 53103), as such a probiotic. The microorganism is particularly employed in a method of preventing or treating food induced hypersensitivity reactions in that it is administered to a recipient together with a food material that has been subjected to a hydrolysis treatment with pepsin and/or trypsin. This Lactobacillus strain selected is described as exhibiting adhesive and colonizing properties and showing a protease enzyme system, so that the protein material contained in the foodstuff to be administered is further hydrolyzed by means of proteases secreted by the specific Lactobacillus strain. The method discussed in this document shall eventually result in the uptake of protein material by the gut that does not show a substantial amount of allergenic material anymore.

EP 0 768 375 further discloses specific strains of the genus Bifidobacterium, that are capable to become implanted in the intestinal flora and may adhere to intestinal cells. These Bifidobacteria are reported to assist in immuno-modulation, being capable to competitively exclude adhesion of pathogenic bacteria to intestinal cells, thus assisting in the maintenance of the individual's health.

During the last few years research has therefore focused on providing additional micro-organisms of this kind all having in common that they are meant to get implanted in the gastro-intestinal microflora, colonizing the gastro-intestinal tract for a certain period of time and exerting their effect in the area or at the location they reside in.

Though probiotics bring about a great deal of beneficial effects for the individual incorporating them a disadvantage resides in that said micro-organisms will only exert their effect when colonizing the gut and secreting the compounds involved in evoking the effect(s). In addition, since the secretion of the respective compound is a biological process, affected by a variety of different parameters, the amount of the compound/metabolite available will vary widely. What is more, since the micro-organisms will colonize only a limited part of the gut which provides favorable conditions for their growth, the effect may be expected to be exerted only there.

Therefore, an object of the present invention resides in obviating the disadvantages of the prior art and providing improved means to deliver substances to an individual beneficial for it.

During the extensive studies leading to the present invention the present inventors have realized that the above object may be solved by applying a novel concept entirely opposite to the notion employed so far.

Thus, according to the present invention the micro-organisms to be used are not meant to survive in the gastro-intestinal tract and colonize it, as is the case with probiotics, but rather the present invention proposes the use of micro-organisms for delivering substances beneficial to the host to specific parts of the gastro-intestinal tract thereof, with the micro-organisms being in a condition ready to release the substance or substances, respectively, in a predetermined location of the gastro-intestinal tract (so-called targeted release).

In order to achieve such a directed delivery and/or release of compounds of interest the micro-organisms utilized will be in a condition that they are going to release their intracellular material at the desired location.

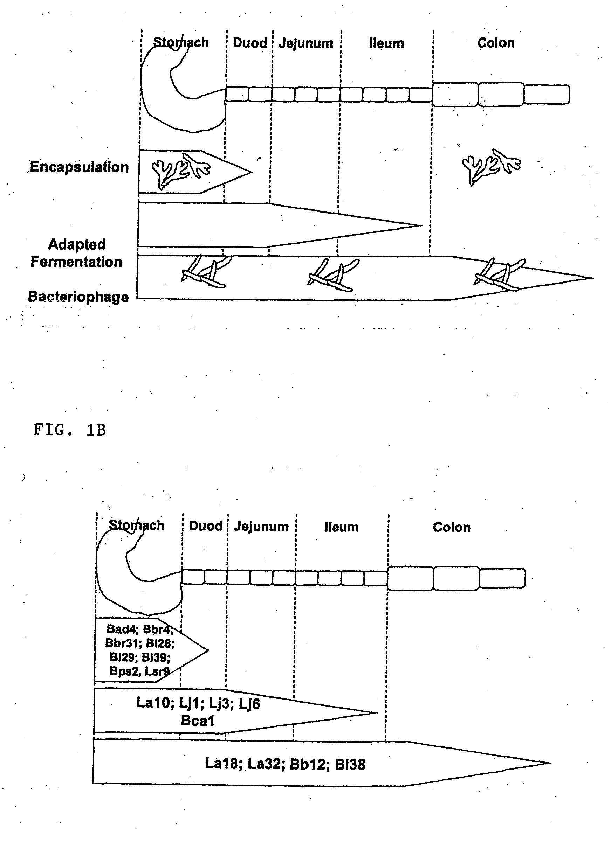

Fig. 1 shows schematically the concept of the present invention, indicating the about area of release and the means to achieve that.

Such a condition, i.e. the capacity to release the biological material of interest at a predetermined location in the gut may be an inherent property of the micro-organism itself, which may result from a sensitivity towards environmental conditions prevailing at said a certain location in the gastro-intestinal tract. In this respect the micro-organism containing the corresponding substance(s) will lyse at a particular location of the gut and will release all of its intracellular material including the substance of interest.

Likewise, such a capacity of releasing the biological material of interest may also result from an appropriate pre-treatment of the micro-organisms prior to administration thereof to a recipient. Such a pretreatment may include weakening the viability of the micro-organisms, so as to reduce their overall growth or endurance performance. On the other hand, such pretreatment may also include enforcing the viability and/or endurance of the corresponding micro-organisms, which may e.g. be achieved by means of encapsulation, so as to e.g. render the micro-organism resistant to gastric and/or bile juices.

For example, an appropriate pretreatment may be effected by subjecting the micro-organisms to a shock treatment, e.g. exposing them to specific conditions of heat, low temperatures or low or high pH-values, respectively, incubating them with phages or inserting recombinant constructs into the micro-organisms which effect an early lysis of the cells, using micro-organisms known to contain prophages, subjecting them to particular fermentative conditions non favorable for growth, triggering a bacterial SOS-response or inactivating the intrinsic protective stress-response systems, which repair damaged intracellular proteins on a regulatory or gene-expression level, so that the resulting micro-organisms will eventually exhibit a reduced viability and/or resistance to environmental conditions/changes in the gut.

It will be appreciated that said reduced viability and/or reduced resistance to environmental conditions/changes will largely depend on the method applied, the sensitivity of the corresponding micro-organisms thereto, and the rate, with which the method had been applied.

As mentioned before, the micro-organisms may also be made more resistant to particular conditions, e.g. by means of encapsulation or triggering a positive and increased intrinsic protective stress-response systems which repairs damaged intracellular proteins on a regulatory or gene-expression level, so that the micro-organisms utilized will sustain for a prolonged time and won't lyse immediately when entering the recipient's gastro-intestinal's tract (e.g. in the stomach with its low pH) and eventually will deliver their freight to a location more distant from the location of entering the recipient's gut (targeted-release).

In principle, the micro-organisms to be used may be any micro-organism not detrimental to the individual to whom they are administered, such as micro-organisms normally used to prepare food material, e.g. lactic acid bacteria, e.g. Lactobacilli, Bifidobacteria, Streptococci, Pediococci, etc. and/or micro-organisms normally colonizing the gastro-intestinal tract, such as Bacteroides, E. coli etc.. According to a preferred embodiment the micro-organisms to be used are selected from the group selected from Lactobacilli and Bifidobacteria and probiotic variants thereof. Preferred examples of such micro-organisms are Bad 4, B128, B129 or La1 all of which are freely available from Depository Institutes under the accession nos. CNCM I-2168, CNCM I-2169, CNCM I-2170 or CNCM I-1225 respectively.

The micro-organisms to be used contain at least one substance the release thereof at the specific location will result in a beneficial effect for the recipient. Such a substance may in principle be any substance known to bring about a desired effect, such as compounds influencing the immune system in an advantageous manner, e.g. by stimulating it or by reducing inflammatory reactions, or substances preventing the growth or overgrowth, respectively, of pathogens in the gastro-intestinal tract to the organisms of the recipient as such, or any substance favoring gut functions such as absorption, motility.

Also envisaged by the present invention are substances designed or known to treat diseases, such as compounds produced by certain micro-organisms, e.g. antibiotics, or other bioactive molecules such as polypeptides, carbohydrates or particular fat.

Examples for such substances are e.g. immuno-modulating compounds, such as e.g. immunoglobulines or interleukines, or compounds, the body may not produce on its own, such as essential fatty acids, compounds known to combat pathogens, such as antimycotika, etc.. Other compounds are bactericidal molecules such as e.g. bacteriocins, autolysins, lipoteichoic acids, lipopolysaccharides, or compounds, the body may not produce on its own, such as essential fatty acids.

Examples for a specific delivery to the small intestine are e.g. substances that interact locally with the mucus layer of the host, aggregate pathogens and facilitate their elimination by mucus flushing substances that enhance nutrient absorption, by complexing specific nutrients and modifying their absorption pattern or substances, e.g. enzymes that have the property to digest pathogen virulence factors (such as enterotoxins).

Examples for a delivery to the colon are e.g. substances that have detoxifying properties, substances that have the potential to control the motility pattern of specific gut portions, substances that have the potential to favor intestinal cell differentiation, substances that have the potential to increase innate immunity or substances that have the potential to restore the mucus layer integrity.

In order to provide a micro-organism containing one or more substances of interest any micro-organism may be selected, that inherently expresses such substances. Since the micro-organisms are designed to release their intracellular material including the beneficial substance(s) at a specific location of the gut, a secretion of the substance into the environment is not required. On the contrary, according to the present invention the substance will be present in higher amounts at the predetermined location, since essentially all of the micro-organism utilized will lyse and release the substance there. To this end, the corresponding micro-organisms already containing the respective substance may optionally be pretreated in a manner appropriate to deliver the substance up to a certain desired location of the gut and may be administered to a recipient, whereupon they will lyse at the respective location in the gut depending on the sort of pretreatment.

This is a great advantage as compared to the common use of probiotics, wherein beneficial substances are primarily released by means of secretion into the environment. According to the present invention the micro-organism utilized will release all of its beneficial cargo essentially at the same time when arriving at the location of the gut, where it is designed to lyse. In addition, the amount of the corresponding substance to be delivered to a recipient may also be more properly controlled, since a given amount of the micro-organism to be used will be administered, with the content of the substance of interest being by and large known.

In order to increase the amount of a given substance to be delivered by the micro-organism common techniques may be used, such as applying particular fermentative conditions or genetically modifying the micro-organism itself, by e.g. subjecting the micro-organisms to a random mutagenesis and selecting those mutants expressing a higher amount of the desired substance. Yet, also recombinant means may be applied, wherein the expression of the endogeneous gene is increased by e.g. linking the corresponding gene with a promotor stronger than the endogenous one, or by inserting the gene or genes encoding the substance(s) of interest into the micro-organism on a plasmid or into the chromosome thereof, optionally linked with a strong promoter that drives the expression of the gene(s) of interest such that the recombinant micro-organism will contain higher amounts of the desired substance.

It will be acknowledged that recombinant techniques will also offer the possibility to insert genes encoding a substance of interest into a micro-organism, that does not inherently contain such a substance. Therefore, any desired substance, that may be expressed by a micro-organism may be included in a given micro-organism by inserting one or more genes encoding said substance(s) into a selected micro-organism and subjecting the recombinant micro-organisms to conditions of culture suitable for expressing the substance(s) and augmenting it (them) in the cell. Following to this, the micro-organisms may be pretreated in a manner suitable for rendering them in a condition delivering the substance to a specific part of the gastro-intestinal tract.

It is also within the concept of the present invention to culture a micro-organism in a broth containing a compound of interest and optionally "treating" the micro-organisms such that they incorporate substances from the medium. Examples for compounds to incorporate in this way are complex carbohydrates or any sort of polypeptides, peptons or proteins.

The micro-organisms of the present invention are therefore to be considered as a vehicle for carrying a substance of interest to a predetermined location or area of the gastro-intestinal tract of a recipient. Since according to the present invention the skilled person may determine, where the substance shall be released, the present invention provides for an excellent means for a directed delivery of a desired substance to specific parts of the gastro-intestinal tract.

Depending on the nature and duration of the pretreatment the endurance of the micro-organism, i.e. its survival in the gastro-intestinal tract may be established, with potential locations of delivery being the mouth, the throat, the stomach, the duodenum, the jejunum, the ileum or the colon. It will be appreciated that the skilled person will, based on his general knowledge and simple experimentation determine the duration of survival of a given micro-organism in the recipient's gut and apply the corresponding treatment before administering the micro-organism to a recipient.

Such micro-organisms are conveniently administered in form of a product acceptable to the consumer, such as an ingestable carrier or support, respectively. Examples for such carriers or supports are a pharmaceutical, galenic or a food composition. Non-limiting examples for such compositions are milk, yogurt, curd, cheese, fermented milks, milk based fermented products, ice-creams, fermented cereal based products, milk based powders, infant formula, pet food, tablets, liquid bacterial suspensions, dried oral supplement, wet oral supplement, dry tube feeding or wet tube feeding.

Such a product may be prepared by choosing a micro-organism, that either already contains the substance(s) to be delivered or that will be modified accordingly in order to provide such (a) substance(s) in a sufficient amount.

The properties of said micro-organism will then be assessed by simple experiments, which show their overall survival in the gastro-intestinal tract. In case of choosing a micro-organism, that is e.g. not resistant to gastric juice, and which should deliver its cargo to a location further downstream in the gastro-intestinal tract, such a micro-organism will preferably be encapsulated in order to ensure its passage through the stomach. On the other hand, when choosing a probiotic micro-organism, that is known to survive the environmental conditions prevailing in the gastro-intestinal tract, and may also start proliferating in the intestine, the viability and/or endurance of such a micro-organism may be weakened such that it does not start proliferating at the desired location but will rather lyse there and deliver its cargo thereto.

Once a micro-organism has been selected and optionally pretreated, said micro-organism may be included in a product as mentioned above in an amount of from 10⁵ - 10¹³ cfu/ml, depending on the nature of the substance to be delivered and the amount of the substance contained in the respective micro-organisms.

The micro-organisms and the above products may consequently be utilized for supporting the well-being of individuals digesting said micro-organism and/or the treatment and/or the prophylaxis of diseases.

The following non-limiting examples further illustrate the invention.

### Example 1

In order to test the sensitivity of different micro-organisms towards the different conditions prevailing in the gastro-intestinal tract, in vitro experiments have been set up, wherein different conditions in the gut were imitated.

Twenty-two bacteria, including 11 *Lactobacillus* strains, 10 *Bifidobacteria* strains and one *Leuconostoc*-strain, originating from the Nestlé culture collection (NCC), were reactivated and initially grown in MRS-broth [Lactobacilli and Leuconostoc] or MRS-Cysteine [Bifidobacteria] **(Table 1)** under anaerobic conditions (anaerobic jar) for 16 hours (24-36 hours for bifidobacteria).

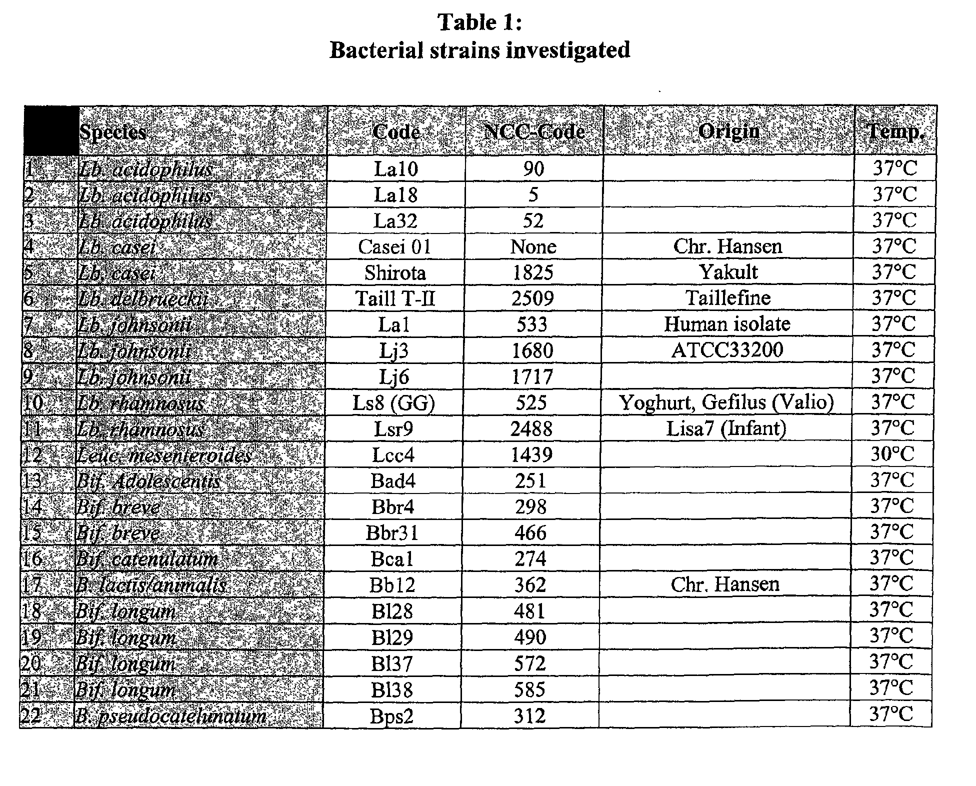

Different physiological test were applied in order to determine the performance and viability of the twenty-two strains (lactobacilli, leuconostoc, bifidobacteria) in simulated gastric juice (SGJ) and bile-salts.
a.) **Resistance to acidic conditions:** 5 ml of simulated gastric juice (0.3% w/v pepsin, 0.5% w/v NaCl, pH 2.0 with HCl) + 1.5 ml 0.5% NaCl were mixed with 1 ml of fresh bacterial culture. After different time-points (0min, 1min, 30 min, 60min, 120min, 180mm) one-ml samples were taken, diluted (serial, tenfold dilutions) and enumerated.
b.) **Resistance to Bile-salts** (Oxgall, Difco): All strains were inoculated in an 1%-inoculum in MRS-broth for 15 hours with different concentrations of bile-acids (0.0%, 0.1%, 0.2%, 0.3%, 0.4%). At the end of this time-period, one-ml samples were taken, diluted (serial, tenfold dilutions) and enumerated.

### The results are summarized in the following tables 2 and 3

As may be derived from table 2 some strains proved to be acid resistant. E.g. all four Lb. rhamnosus strains (incl. Ls8 = Lb. GG) are already loosing one log (+/- 0.2 logs) of bacterial counts (cfu/ml )after 1 min exposure to the simulated-gastric juice (SGJ). After 30 min, a reduction of at least 2.4 logs (and up to 5 logs) could be observed for all four Lb. rhamnosus strains. After 60 min. exposure to the SGJ, all bacteria are basically dying off (loss of at least 6 logs). In this context, it became clear that all investigated Lb. rhamnosus strains are very sensitive to low pH.

Lb. johnsonii Lj3 is loosing about 0.6 to 1.0 logs after an exposure of 1 min to the SGJ. An additional exposure for 30, 60, 120 and 240 min did not reduce the viable bacterial numbers further. In this context it becomes quite obvious that the tested Lb. johnsonii strain is very stable against low pH. This finding has been observed earlier for Lb. johnsonii La1 (NCC533).

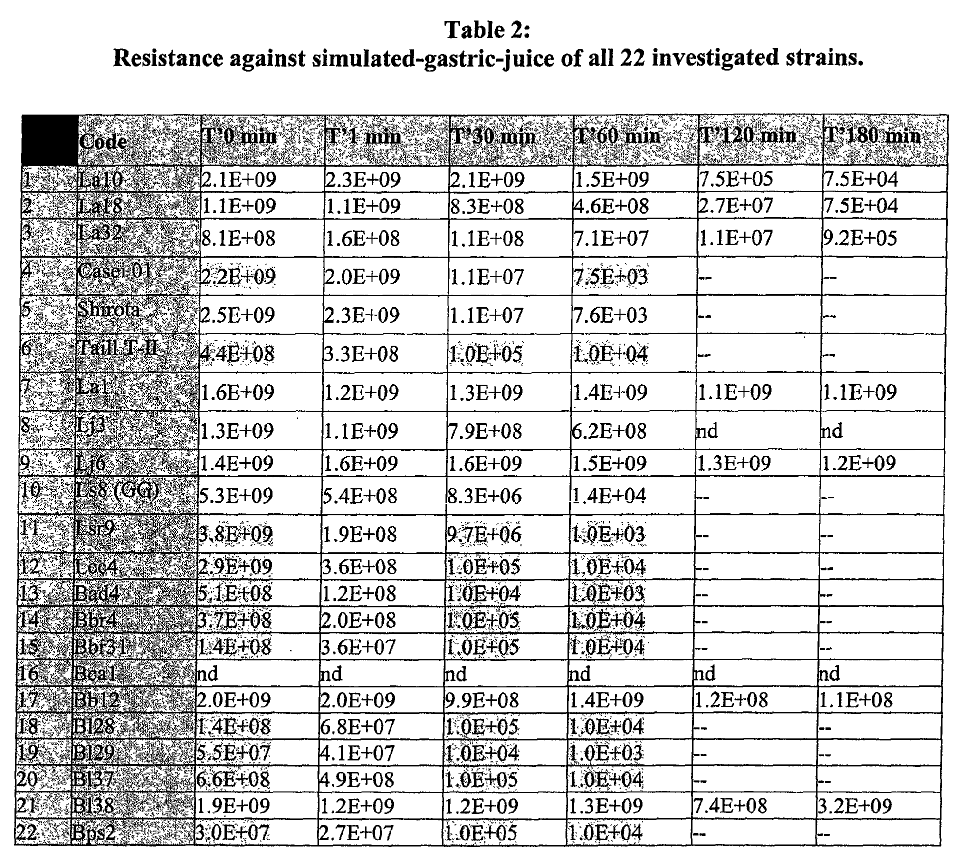

The highlighted strains have been found not to be stable at pH 2.0 and are considered good candidates for a rapid lysis and a targeted release of desired compounds in the stomach.

In table 3 the results of the experiments in the bile salts are summarized. The strains obviously were behaving quite different in this challenge-test against bile-salts:

All Lb. rhamnosus/casei isolates were generally rather stable to different concentrations of bile-salts. A concentration of 0.1% reduced bacterial counts maximal 0.6 logs. Raising the concentration to 0.2% resulted in a loss between 0.5 and 0.9 logs. At 0.3% Oxgall, reduction was generally in the range of one log (+/- 0.2 logs), whereas a concentration of 0.4% bile-salts led to a diminuation of viable bacteria after 17 hours exposure of 1.0 to 1.7 logs, depending which strain was investigated. Lb. rhamnosus Ls8 (=Lb. GG) is rather stable against bile-salts, resulting in an overall loss of only one log, which is very significant.

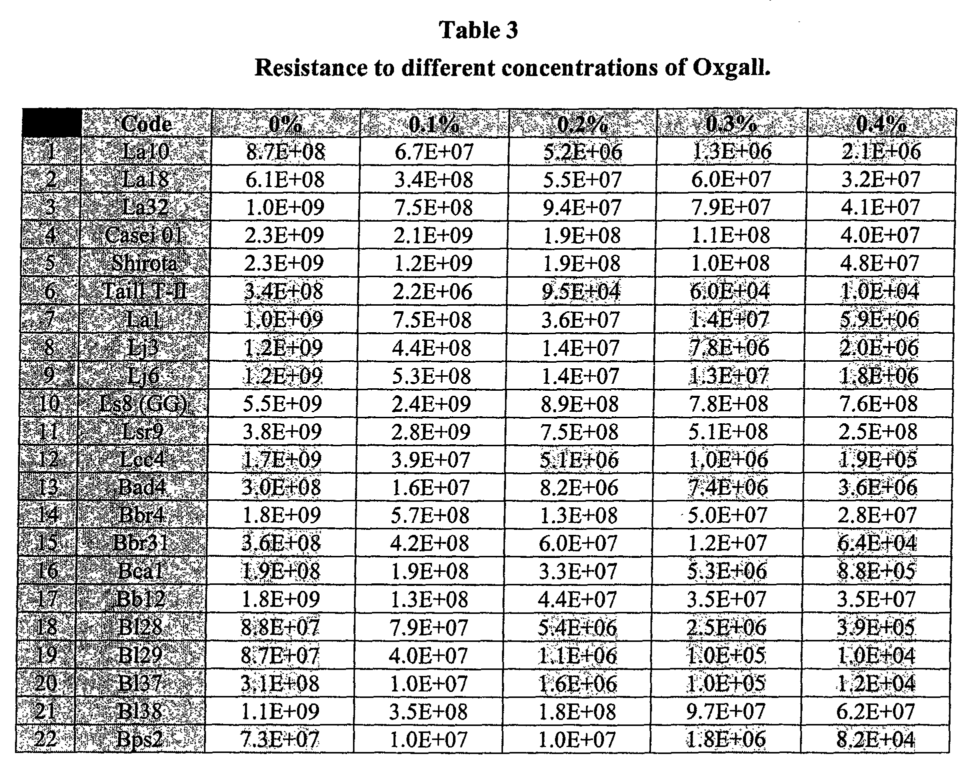

All bacterial strains were inoculated at 1% in MRS and grown at 37°C or 30°C for 16 h.

The highlighted strains are not stable to various concentrations of bile salts and are good candidates for a rapid lysis and a targeted release at this location of the intestinal tract

It will be appreciated that the skilled person may well examine other strains for their aptitude to lyse at a certain location in the gastro-intestinal tract, by subjecting them to the condtions as detailed above or others, reflecting an environment as found in the gut.

## Claims

1. Use of a micro-organism for delivering substances beneficial to the host to a specific part of the gastro-intestinal tract, wherein the micro-organism is in a condition, that it releases said substance in a predetermined location of the gut.

2. The use according to claim 1, wherein the micro-organism is sensitive to environmental conditions prevailing in the specific part of the gastro-intestinal tract, where it delivers the compounds to.

3. The use according to claim 2, wherein the micro-organism has been pretreated such that it ceases to subsist particular environmental conditions prevailing in the part of the gastro-intestinal tract, where it delivers the compounds to.

4. The use according to claim 3, wherein the micro-organism has been pretreated by means of exposure to heat, to cold, to different pH-values, to phages, encapsulation by proteins, carbohydrates, fat or any other organic and inorganic substance, subjected to particular fermentative conditions or triggering a bacterial SOS-response or inactivating the intrinsic protective stress-response systems which repair damaged intracellular proteins on a regulatory or gene-expression level.

5. The use according to any of the preceding claims, wherein the micro-organism is selected from the group consisting of Lactobacilli, Bifidobacteria, Streptococci, Pediococci, Enterococci, Lactococci, Oenococci, Staphylococci, Bacteroides, or mixtures thereof.

6. The use according to any of the preceding claims, wherein the micro-organism is a probiotic.

7. The use according to any of the preceding claims, wherein the micro-organism is a modified micro-organism, carrying one or more genes yielding at least one substance beneficial to the host.

8. The use according to claim 7, wherein the one or more genes yielding at least one substance beneficial to the host are modified such that a higher expression thereof results or wherein the one or more genes have been introduced in said micro-organism from an exogenous source.

9. The use according to any of the preceding claims, wherein the micro-organism ceases to subsist in the stomach, the duodenum, the jejunum, the ileum or the colon.

10. The use according to any of the preceding claims, wherein the substance to be delivered is selected from the group consisting of peptides, peptones, proteins, monosaccharides, disaccharides, complex carbohydrates or fat.

11. An ingestable product containing a micro-organism to be used for delivering substances beneficial to the host to specific parts of the gastro-intestinal tract thereof.

12. The ingestable product, according to claim 11, which is a pharmaceutical, a galenic or a food product.

13. The product according to claim 12, which is selected from the group consisting of milk, yogurt, curd, cheese, fermented milks, milk based fermented products, ice-creams, fermented cereal based products, milk based powders, infant formula, pet food, tablets, liquid bacterial suspensions, dried oral supplement, wet oral supplement, dry tube feeding or wet tube feeding.

14. A process for the preparation of an ingestable product according to any of the claims 11 to 13, which comprises, providing a micro-organism containing a substance beneficial for the host, optionally pretreating said micro-organisms in a manner to ensure that it will deliver the substance(s) of interest to the selected part of the gut, and including said micro-organism in an appropriate carrier.

15. Use of a micro-organism, capable of delivering substances beneficial to the host to specific parts of the gastro-intestinal tract thereof, for supporting the well-being of individuals digesting said micro-organism(s) and/or the treatment and/or the prophylaxis of diseases.
